Europäisches Patentamt

European Patent Office

Office européen des brevets

(11) Publication number: **0 408 004 A1**

# EUROPEAN PATENT APPLICATION

(21) Application number: **90113263.9**

(22) Date of filing: **11.07.90**

(51) Int. Cl.⁵: **C07C 19/08, C07C 17/00**

(30) Priority: **12.07.89 IT 2116089**

(43) Date of publication of application:
**16.01.91 Bulletin 91/03**

(84) Designated Contracting States:
**BE CH DE ES FR GB LI NL SE**

(71) Applicant: **AUSIMONT S.r.l.**
**31, Foro Buonaparte**
**I-20100 Milano(IT)**

(72) Inventor: **Cuzzato, Paolo**
**6, Via Boccaccio**
**I-31100 Treviso(IT)**
Inventor: **Masiero, Antonio**
**29/4 Via Marchesi**
**I-35048 Stanghella, Padova(IT)**

(74) Representative: **Barz, Peter, Dr. et al**
**Patentanwälte Dipl.-Ing. G. Dannenberg Dr.**
**P. Weinhold, Dr. D. Gudel Dipl.-Ing. S.**
**Schubert, Dr. P. Barz Siegfriedstrasse 8**
**D-8000 München 40(DE)**

(54) **Process for preparing 1,2-dichloro-1,1,2-trifluoroethane.**

(57) 1,2-Dichloro-1,1,2-trifluoroethane is prepared, in high yields, by reacting tetrachloroethylene and hydrofluoric acid in the gas phase and in the presence of a catalyst comprising a chromium salt, for example $CrCl_3$, carried on $AlF_3$ having a specific surface area of at least 25 $m^2/g$, preferably at temperatures ranging from 220 to 280° C.

## PROCESS FOR PREPARING 1,2-DICHLORO-1,1,2-TRIFLUOROETHANE

The present invention relates to a process for preparing 1,2-dichloro-1,1,2-trifluoroethane by catalytic reaction, in the gas phase, of tetrachloroethylene and hydrofluoric acid.

US-A-4 766 260 discloses that 1,2-dichloro-1,1,2-trifluoroethane may be obtained as a by-product in the reaction for the preparation of 1,1,1-trifluorodichloroethane and 1,1,1,2-tetrafluorochloroethane by reacting tetrachloroethylene and hydrofluoric acid in the presence of catalysts consisting of Ni, Cr, Mn or Co compounds carried on a composition comprising $Al_2O_3$ and major amounts of $AlF_3$.

In said reaction, the amount of 1,2-dichloro-1,1,2-trifluoroethane which forms as by-product does not exceed 5% by weight of the reaction products obtained.

A process, which is the object of the present invention has now, surprisingly, been found for preparing 1,2-dichloro-1,1,2-trifluoroethane in high yields. Said process comprises the reaction of tetrachloroethylene and hydrofluoric acid in the gas phase and in the presence of a catalyst comprising a trivalent chromium salt supported on $AlF_3$ having a specific surface area of at least 25 $m^2$/g, and preferably ranging from 25 $m^2$/g to 40 $m^2$g.

Preferably, the reaction is conducted at a temperature of from 220 to 280° C, particularly from 240 to 250° C. The preferred contact times of the reagents in the reactor, under said conditions, range from 5 to 30 seconds, particularly from 10 to 20 seconds.

The present process is highly efficient as the by-products, other than the dichlorotrifluoroethane isomers, are almost completely composed of intermediates of the synthesis of said $C_2HCl_2F_3$ and, thus, can be recycled to the reactor along with the unconverted tetrachloroethylene, thereby realizing a continuous process, with recycle, which offers evident economic advantages.

The catalyst employed in the process of the present invention may be prepared by repeatedly impregnating aluminium fluoride with an aqueous solution of the chromium salt and by drying each time until obtaining on the carrier, in the dry state, an amount of chromium, calculated as metal, of at least 8% by weight, based on the total weight of the supported catalyst.

$CrCl_3$ is preferably used as chromium compound.

The following examples merely serve to illustrate the invention and do not limit the scope thereof.

Example 1

Catalyst Preparation

A tubular Inconel reactor having a diameter of 8 cm and a length of 100 cm, electrically heated and equipped with a sintered porous Inconel baffle, was charged with 1680 g of a catalyst, prepared as described below.

A carrier consisting of $AlF_3$, predominantly in the gamma-form and having a specific surface area of 26 $m^2$/g, was impregnated with an aqueous solution of $CrCl_3 \cdot 6H_2O$ in an amount of 492 g of $CrCl_3 \cdot 6H_2O$ per kg of $AlF_3$.

Said solution was composed of 492 g of $CrCl_3 \cdot 6H_2O$ in 152 ml of $H_2O$, had a volume of 450 ml and was added to the $AlF_3$ in three almost equal portions. After each addition, the catalyst was dried for 4 hours at 120° C and atmospheric pressure.

After the third drying operation, the catalyst was sieved and placed into the reactor.

The catalyst was fluidized with a nitrogen stream (about 100 l/h) and heated at 400° C for 10 hours, whereafter the reactor was brought to the operating temperature.

Example 2

0.87 moles/h of $C_2Cl_4$ and 4.29 moles/h of anhydrous HF were introduced, at a pressure slightly higher than atmospheric pressure and at a temperature of 260° C, into the reactor described above, thereby obtaining a contact time of 19.9 seconds, calculated as the ratio of non-fluidized catalyst volume to volumetric flow rate of the reagents fed at the reaction temperature and pressure.

The gases leaving the reactor were collected for 1 hour. After absorption of HCl and HF in water and washing of the reaction product with an aqueous solution of NaOH, 143 g of product were recovered, the molar composition of which was as follows:

| | |
|---|---|
| $CF_2Cl\text{-}CHFCl$ | 1.3% |
| $CF_3\text{-}CHCl_2$ | 7.7% |
| $C_2Cl_4$ | 77.8% |

the balance mainly consisting of $CCl_2 = CCIF$ and $CCIF_2\text{-}CHCl_2$. The $C_2Cl_4$ conversion was 22.2% and the percentage of 1,1,2-trifluoro-1,2-trichloroethane, based on the total amount of $C_2HCl_2F_3$ was 14.5%.

Example 3

The reactor of example 2 was charged, at 280° C and at a pressure slightly higher than atmospheric pressure, with 0.82 moles of $C_2Cl_4$ and 4.13 moles of HF, thereby obtaining a contact time of 20 seconds.

Operating according to example 2, 128 g of products were obtained, the molar composition of which was as follows:

| | |
|---|---|
| $CF_3\text{-}CHF_2$ | 4.7% |
| $CF_3\text{-}CHFCl$ | 14.1% |
| $CF_2Cl\text{-}CHFCl$ | 1.0% |
| $CF_3\text{-}CHCl_2$ | 23.6% |
| $C_2Cl_4$ | 49.5% |

the balance mainly consisting of $CCIF_2\text{-}CHCl_2$, $CCl_2 = CCIF$ and $CF_3\text{-}CH_2Cl$.

The conversion of $C_2Cl_4$ was 50.5% and the percentage of 1,1,2-trifluoro-1,2-dichloroethane, based on the total amount of $C_2HCl_2F_3$, was 4.1%.

Example 4

The reactor described above was charged, at 240° C and at a pressure slightly higher than atmospheric pressure, with 1.75 moles of $C_2Cl_4$ and 8.60 moles of HF, thereby obtaining a contact time of 10 seconds.

Operating as in example 2, 290 g of a product were obtained, the molar composition of which was as follows:

| | |
|---|---|
| $CF_2Cl\text{-}CHFCl$ | 0.7% |
| $CF_3\text{-}CHCl_2$ | 2.8% |
| $C_2Cl_4$ | 89.5% |

the balance mainly consisting of $CCIF_2\text{-}CHCl_2$ and $CCl_2 = CCIF$. The conversion of $C_2Cl_4$ was 10.5% and the percentage of 1,1,2-trifluoro-1,2-dichloroethane, based on the total amount of $C_2HCl_2F_3$, was 20.0%.

**Claims**

1. Process for preparing 1,2-dichloro-1,1,2-trifluoroethane, which comprises reacting tetrachloroethylene and hydrofluoric acid in the gas phase and in the presence of a catalyst comprising a trivalent chromium salt supported on aluminium trifluoride having a specific surface area of at least 25 $m^2/g$.

2. Process according to claim 1, wherein the reaction is carried out at a temperature of from 220 to 280° C.

3. Process according to any one of claims 1 and 2, wherein the reaction is conducted with contact times of the reagents of from 5 to 30 seconds.

4. Process according to any one of claims 1 to 3, wherein the chromium salt comprises chromium

trichloride.

## DOCUMENTS CONSIDERED TO BE RELEVANT

EP 90113263.9

| Category | Citation of document with indication, where appropriate, of relevant passages | Relevant to claim | CLASSIFICATION OF THE APPLICATION (Int. Cl.5) |
|---|---|---|---|
| A | <u>DE - B2 - 1 910 529</u> (DYNAMIT NOBEL) * Claims 1-3 * | 1-4 | C 07 C 19/08 C 07 C 17/00 |
| D,A | <u>US - A - 4 766 260</u> (MANZER et al.) * Column 3, line 63; examples 10-13 * | 1,3,4 | |
| A | <u>EP - A1 - 0 282 005</u> (AUSIMONT) * Examples 1,2 * | 1,3,4 | |
| A | <u>EP - A1 - 0 308 923</u> (DAIKIN INDUSTRIES) * Examples 1-3 * | | |

TECHNICAL FIELDS
SEARCHED (Int. Cl.5)

C 07 C 19/00
C 07 C 17/00

The present search report has been drawn up for all claims

| Place of search | Date of completion of the search | Examiner |
|---|---|---|
| VIENNA | 04-10-1990 | KÖRBER |

EPO FORM 1503 01.82 (P0401)